(19) Europäisches Patentamt
European Patent Office
Office européen des brevets

(11) Numéro de publication: **0 472 966 B1**

(12) **FASCICULE DE BREVET EUROPEEN**

(45) Date de publication de fascicule du brevet: **28.09.94**

(51) Int. Cl.$^5$: **C07C 69/02**, A61K 7/46, C07C 43/13

(21) Numéro de dépôt: **91113240.5**

(22) Date de dépôt: **07.08.91**

# (54) Esters nouveaux et leur utilisation en parfumerie.

(30) Priorité: **28.08.90 CH 2799/90**

(43) Date de publication de la demande: **04.03.92 Bulletin 92/10**

(45) Mention de la délivrance du brevet: **28.09.94 Bulletin 94/39**

(84) Etats contractants désignés: **CH DE FR GB LI NL**

(56) Documents cités:
**DE-A- 2 015 865**
**US-A- 2 410 008**
**US-A- 4 504 412**

(73) Titulaire: **FIRMENICH SA**
**1, route des Jeunes**
**CH-1211 Genève 8 (CH)**

(72) Inventeur: **Giersch, Wolfgang Klaus**
**323, rue de Bernex**
**CH-1233 Bernex (CH)**
Inventeur: **Schulte-Elte, Karl-Heinrich**
**44, chemin de Carabot**
**CH-1213 Onex (CH)**
Inventeur: **Mahaim, Cyril**
**Route du Village B**
**CH-1112 Echichens (CH)**

(74) Mandataire: **Salvadori, Giuseppe, Dr.**
**c/o Firmenich S.A.**
**Case Postale 239**
**CH-1211 Genève 8 (CH)**

EP 0 472 966 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait à la parfumerie. Elle concerne, plus particulièrement, des composés de formule

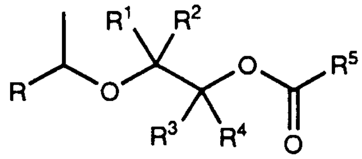

(I)

dans laquelle le symbole R représente un radical 3,3-diméthyl-1-cyclopentyle ou 3,3-diméthyl-1-cyclohexyle, le symbole $R^5$ représente un radical alkyle saturé de $C_1$ à $C_3$, de chaîne linéaire ou ramifiée et, soit $R^1$ et $R^2$ représentent chacun un atome d'hydrogène, $R^3$ et/ou $R^4$ représentant un radical méthyle, soit $R^3$ et $R^4$ représentent chacun un atome d'hydrogène, $R^1$ et/ou $R^2$ représentant alors un radical méthyle.

Les esters de formule (I) sont des composés nouveaux. Nous avons découvert qu'ils possèdent des propriétés odorantes très utiles et qu'ils peuvent, de ce fait, être employés pour la préparation de compositions parfumantes et d'articles parfumés. Ils servent, en effet, à développer des notes odorantes de type musqué, ambrette, accompagnées de caractères de type fruité et parfois floral.

Les composés de l'invention peuvent se présenter soit à l'état isolé soit sous forme d'un mélange isomérique et ce en fonction de la nature des substituants $R^1$ à $R^4$. Ainsi les composés (I) peuvent se présenter sous forme d'un mélange d'isomères de formule

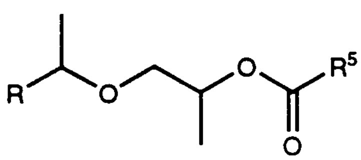

et

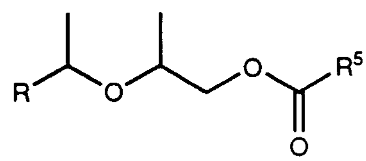

(Ia) (Ib)

ou sous forme d'un mélange d'isomères de formule

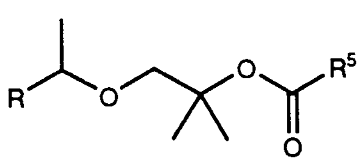

et

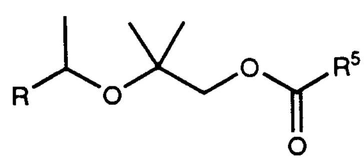

(Ic) (Id)

dans lesquelles R et $R^5$ sont définis comme à la formule (I).

L'invention a également trait à ces mélanges d'isomères. Nous avons en effet constaté que ces mélanges constituaient aussi des ingrédients parfumants très avantageux, d'un emploi alternatif à celui de leurs ingrédients individuels.

Parmi les composés de formule (Ia) à (Id), les composés de formule (Ib) ou (Id) sont cités à titre préférentiel, leur odeur possédant un caractère musc-ambrette plus prononcé et plus net et élégant que celle des composés (Ia) ou, respectivement (Ic).

Par ailleurs, il a également été constaté que, malgré leur caractère de base musc-ambrette commun, les isomères optiquement actifs des composés préférés (Ib) ou (Id) possédaient des odeurs distinctes, certains isomères pouvant être plus performants que d'autres.

Parmi les composés de l'invention il y a lieu de citer à titre préférentiel le propionate de 4-(3,3-diméthyl-1-cyclohexyl)-2,2-diméthyl-3-oxapentyle. Ce composé possède une odeur de type musqué, ambrette, avec un net côté fleuri et un caractère fruité de type poire. Cette combinaison de caractères ambrette et poire est particulièrement intéressante et caractéristique de la série de composés (I), lesquels

confèrent aux compositions dans lesquelles ils sont intégrés des notes de type poire et graines d'ambrette tout à fait surprenantes, comme il ressort des exemples d'application présentés plus loin.

L'effet olfactif imparti par le composé préféré de l'invention cité ci-dessus était encore plus puissant et plus musqué lorsque ce composé était utilisé sous forme de l'un de ses isomères optiquement actifs, à savoir le composé de formule

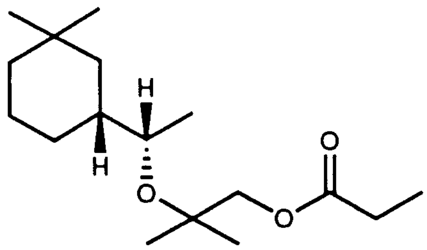

(Ie)

ou propionate de (+)-(1'R,4S)-4-(3',3'-diméthyl-1'-cyclohexyl)-2,2-diméthyl-3-oxapentyle. Cependant, les autres isomères optiquement actifs de ce composé étaient également des ingrédients parfumants utiles. De même, les mélanges du propionate de 4-(3,3-diméthyl-1-cyclohexyl)-2,2-diméthyl-3-oxapentyle avec le propionate de 4-(3,3-diméthyl-1-cyclohexyl)-1,1-diméthyl-3-oxapentyle étaient également des ingrédients parfumants utiles pour les applications selon l'invention et moins onéreux que le composé pur préféré.

On peut également citer à titre de composés préférés selon l'invention, le propionate de 4-(3,3-diméthyl-1-cyclohexyl)-2-méthyl-3-oxapentyle et le propionate de 4-(3,3-diméthyl-1-cyclohexyl)-1-méthyl-3-oxapentyle, ainsi que leurs mélanges. Ces compositions chimiques possèdent des notes odorantes de type musqué, ambrette, avec un net côté poire William. Le propionate de 4-(3,3-diméthyl-1-cyclohexyl)-2-méthyl-3-oxapentyle est l'isomère préféré du point de vue olfactif.

Les propriétés odorantes des autres composés selon l'invention seront décrites en détail dans les exemples de préparation respectifs présentés plus loin.

Les composés et mélanges selon l'invention peuvent être utilisés aussi bien en parfumerie fine, pour la préparation de compositions et bases parfumantes, parfums et eaux de toilette, qu'en parfumerie fonctionnelle, pour le parfumage d'articles de consommation divers. Dans ce cas, on peut citer à titre d'exemple les savons, les gels de bain ou douche, les shampoings ou autres produits de traitement capillaire, les désodorisants corporels ou d'air ambiant. Ils sont également utiles pour le parfumage de détergents ou revitalisants textiles et de produits d'entretien.

Dans ces applications, les composés de l'invention peuvent être utilisés seuls ou en mélange avec d'autres coingrédients parfumants, ainsi que des solvants ou supports usuels en parfumerie. Les concentrations dans lesquelles ils sont utilisés peuvent varier dans une gamme de valeurs très étendue. L'homme du métier sait par expérience que ces valeurs sont fonction de l'effet parfumant désiré, ainsi que de la nature des autres ingrédients présents dans une composition donnée. A titre d'exemple, on peut citer des concentrations de l'ordre de 5 à 10% en poids, voire même 20% ou plus, du composé ou du mélange selon l'invention, par rapport au poids de composition. Ces valeurs peuvent être nettement inférieures lorsque les composés selon l'invention sont utilisés pour le parfumage des articles fonctionnels susmentionnés.

Les composés de formule (I) ou leurs mélanges selon l'invention sont préparés selon un procédé original caractérisé en ce qu'on traite avec un agent d'estérification approprié un hydroxy-éther de formule

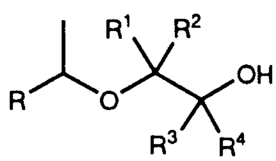

(II)

dans laquelle les symboles R et $R^1$ à $R^4$ ont le sens indiqué à la formule (I), ou un mélange d'isomères structurels de formule (II).

Les hydroxy-éthers de formule (II), utilisés comme produits de départ dans le procédé de l'invention, peuvent être obtenus à partir des éthanones appropriées selon le procédé décrit schématiquement ci-dessous. Dans ce schéma, les symboles R et $R^1$ à $R^4$ ont le sens indiqué à la formule (I).

## SCHEMA I

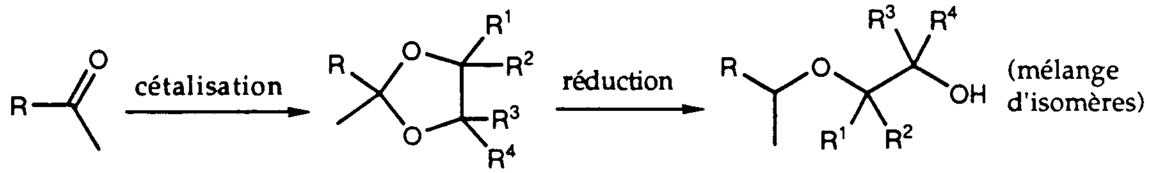


( II )

Les éthanones de départ dans ce schéma réactionnel, à savoir les 1-(3,3-diméthyl-1-cyclohexyl)-1-éthanone ou cycladémone et 1-(3,3-diméthyl-1-cyclopentyl)-1-éthanone, peuvent être facilement obtenues, dans le premier cas par des procédés connus [voir, par exemple, J.B. Hall et al., J. Org. Chem. 37,920(1972); H.R. Ansari, Tetrahedron 29,1559(1973)] et, pour ce qui est de l'éthanone citée en deuxième lieu, à partir de 4,4-diméthyl-1-cyclopentène-1-carbaldéhyde, comme il est décrit plus loin.

Les réactions représentées dans ce schéma sont des réactions de type classique dont les conditions spécifiques sont décrites en détail dans les exemples de préparation présentés plus loin. L'étape de réduction du cétal intermédiaire représenté conduit à un mélange d'isomères de formule (II) qui peuvent être ensuite séparés à l'aide de techniques de séparation usuelles telles que la chromatographie en phase préparative.

L'estérification de ces composés, qui s'effectue dans les conditions décrites en détail plus loin, permet d'obtenir les composés de formule (I) désirés.

Alternativement, le mélange d'isomères (II) susmentionné peut être transformé, selon le procédé de l'invention, en un mélange de composés isomères de formule (I). Ce mélange peut être utilisé tel quel dans les applications en parfumerie selon l'invention, comme il a été cité précédemment.

Les composés de formule (II) peuvent également être obtenus selon un procédé alternatif représenté schématiquement ci-dessous :

## SCHEMA II

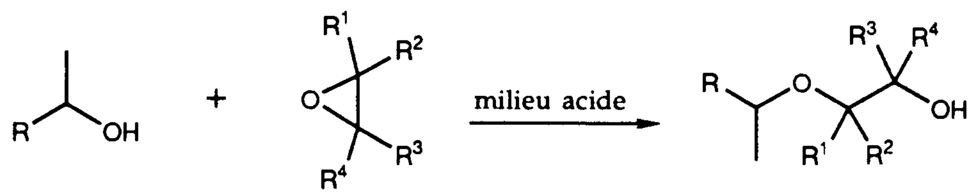


( II )

Dans ce schéma les symboles R et $R^1$ à $R^4$ ont le sens indiqué à la formule (I). Le procédé illustré au schéma II se révèle être plus avantageux que celui représenté au schéma I, car il permet d'obtenir sélectivement l'un des isomères de formule (Ia) ou (Ib), rendant ainsi superflue l'étape de séparation des deux isomères.

L'alcool de départ dans cette méthode peut être obtenu de façon classique à partir de la cétone de départ représentée au schéma I ou selon d'autres procédés comme il est décrit plus loin.

La réaction de cet alcool avec l'époxyde représenté est effectuée en milieu acide, par exemple dans un acide de type de Lewis. Les conditions spécifiques de ces réactions sont décrites plus loin dans les exemples de préparation.

L'utilisation de l'alcool de départ sous une forme isomérique optiquement active appropriée permet, bien entendu, d'obtenir les composés (I) sous des formes optiquement actives correspondantes, comme il est décrit plus loin.

L'invention sera maintenant décrite plus en détail à l'aide des exemples de préparation présentés ci-après, dans lesquels les températures sont indiquées en degrés centigrades et les abréviations ont le sens usuel dans l'art.

L'invention sera également illustrée à l'aide d'exemples d'application en parfumerie des composés et mélanges selon l'invention.

Exemple 1

Préparation d'acétate de 4-(3,3-diméthyl-1-cyclohexyl)-2-méthyl-3-oxapentyle et d'acétate de 4-(3,3-diméthyl-1-cyclohexyl)-1-méthyl-3-oxapentyle

a) Préparation d'un mélange des deux isomères structurels (en un pot)

Dans un réacteur de type Schmizo de 4 l, muni d'une agitation mécanique, d'un séparateur d'eau, d'un réfrigérant, d'un thermomètre et d'une ampoule d'introduction, on a chargé 200 g (1,3 mole) de cycladémone, 197,6 g de 1,2-propanediol, 500 ml de toluène et 2 g d'acide p-toluène-sulfonique. On a chauffé à reflux pendant 8 $_{1/2}$ h en séparant l'eau et une partie de l'excès du diol qui distille aussi azéotropiquement avec le toluène. Après refroidissement à température ambiante, on a lavé avec 300 ml de saumure, décanté, ajouté 100 ml de toluène et chauffé à reflux pendant 3 h avec un séparateur d'eau pour enlever le reste d'eau et de diol.

On a ensuite chauffé à 80° et introduit lentement 1300 ml de solution d'hydrure de diisobutylaluminium (DIBAH) dans le toluène (1,95 mole, 1,5 M). La réaction était fortement exothermique au départ (> 100°). L'introduction a été accélérée, en maintenant la température à ≈ 90°, et complétée en 2 h. On a agité pendant un quart d'heure à 100°.

Après refroidissement à température ambiante, on a introduit 220 ml (2,16 mole) d'anhydride acétique. Après avoir introduit 20 ml de produit, la réaction a cessé d'être exothermique et le restant de l'anhydride a été ajouté pendant qu'on chauffait à 60° (2 h). Le mélange réactionnel a ensuite été chauffé à reflux (≈ 110°) pendant 3 h. Après refroidissement, on a versé sur glace, ajouté 2 l de HCl à 10% et 2 l d'éther de pétrole 30/50°. On a neutralisé à la saumure, repris les eaux à l'éther de pétrole, séché sur $Na_2SO_4$, filtré et concentré au rotavapeur pour obtenir 330,3 g de produit brut. Ce dernier a été distillé sur résidu pour fournir 301,6 g d'un mélange contenant les deux acétates désirés.

P.éb. 100°/4 Pa ; rend.: 90,6%

Propriétés odorantes : ce mélange possédait une faible odeur musquée, avec un côté graine d'ambrette, poire très naturel; curieusement, le mélange était pratiquement inodore en tête et nettement plus puissant en fond.

b) Préparation des isomères individuels

On a préparé un mélange de 4-(3,3-diméthyl-1-cyclohexyl)-2-méthyl-3-oxa-1-pentanol et 5-(3,3-diméthyl-1-cyclohexyl)-4-oxa-2-hexanol, à partir de la cycladémone, comme il est décrit dans les deux premiers paragraphes de a). Ce mélange a ensuite été oxydé, l'alcool primaire étant transformé en acide et l'alcool secondaire en cétone, permettant ainsi une séparation chimique de ces deux produits qui peuvent ensuite être traités séparément pour produire les acétates désirés.

On a procédé ainsi : à une solution de 43 g de chlorochromate de pyridinium (PCC) dans 50 ml de diméthyl formamide (DMF), on a ajouté sous agitation et goutte à goutte, une solution de 7,5 g du mélange d'alcools susmentionné dans 50 ml de DMF. On a laissé réagir pendant 15 h. On a versé sur 900 ml d'eau et extrait 2 fois avec 500 ml d'éther éthylique

Les phases organiques combinées, contenant la 5-(3,3-diméthyl-1-cyclohexyl)-4-oxa-2-hexanone, ont été lavées 4 fois avec une solution diluée de NaOH. La phase organique a été concentrée et distillée pour fournir 1,2 g de cétone.

P.éb. 150° (bain)/10 Pa

RMN($^1$H,360MHz) : 2,1(s,C$\underline{H}_3$C = O) δ ppm.

Les eaux alcalines combinées, mélangées avec de la glace, ont été acidifiées et les acides ainsi formés ont été extraits à l'éther. Après distillation au four à boules, on a obtenu 3 g d'acide 4-(3,3-diméthyl-1-cyclohexyl)-2-méthyl-3-oxapentanoïque.

P.éb. 170° (bain)/6 Pa

RMN($^1$H,360MHz) : 10,1(s,C(O)O$\underline{H}$) δ ppm

Un mélange de la cétone susmentionnée avec 0,1 g de $LiAlH_4$ dans l'éther éthylique a été chauffé à reflux pendant 2 h. Après refroidissement, on a ajouté 0,1 ml d'eau, 0,1 ml de NaOH à 15% et 0,3 ml d'eau. On a filtré et concentré pour obtenir 1,0 g de 5-(3,3,-diméthyl-1-cyclohexyl)-4-oxa-2-hexanol. Ce dernier a été traité, sous agitation, avec 1 ml d'anhydride acétique et 2 ml de pyridine, pendant 15 h à température ambiante. Après avoir concentré et chromatographié, on a obtenu 0,15 g de produit contenant 90% d'acétate de 4-(3,3-diméthyl-1-cyclohexyl)-1-méthyl-3-oxapentyle et environ 10% de l'acétate primaire isomère.

Un traitement similaire de l'acide oxapentanoïque susmentionné a fourni 2 g d'acétate de 4-(3,3-diméthyl-1-cyclohexyl)-2-méthyl-3-oxapentyle à l'état pur.

Données analytiques :

acétate de 4-(3,3-diméthyl-1-cyclohexyl)-1-méthyl-3-oxapentyle
(4 stéréoisomères)

RMN($^1$H,360MHz) : 0,87(s,3H) ; 0,90(s,3H) ; 1,07(d,J=6Hz,3H) ; 1,23(d,J=6Hz,3H) ; 2,05(s,3H) ; 3,07(m,1H) ; 3,34(m,1H) ; 3,5(m,1H) ; 5,02(m,1H) δ ppm

SM : 256($M^+$,0), 139, 138, 123, 101, 83, 69, 55, 43

acétate de 4-(3,3-diméthyl-1-cyclohexyl)-2-méthyl-3-oxapentyle
(4 stéréoisomères)

RMN($^1$H,360MHz) : 0,88(s,3H) ; 0,91(s,3H) ; 1,07 et 1,15(d,6H) ; 2,07(s,3H) ; 3,16(m,1H) ; 3,66-(m,1H) ; 4,0(m,2H) δ ppm

SM : voir ci-dessus.

Propriétés odorantes : comme c'était le cas avec leur mélange décrit en a), ces deux isomères développent des notes de fond beaucoup plus puissantes que leurs notes de tête. Il s'agit de notes de la même qualité que celle du mélange mais, dans le cas de l'acétate primaire, plus puissante que la note du mélange. Cette dernière possède aussi un caractère ambrette plus prononcé et est plus élégante dans le contexte floral.

Exemple 2

Préparation de propionate de 4-(3,3-diméthyl-1-cyclohexyl)-2-méthyl-3-oxapentyle et de propionate de 4-(3,3-diméthyl-1-cyclohexyl)-1-méthyl-3-oxapentyle

Dans un réacteur équipé d'un séparateur d'eau, on a chargé 500 g (3,2 mole) de cycladémone, 304 g (4 mole) de 1,2-propanediol, 3 l d'éther de pétrole 80/100° et 2 g d'acide p-toluènesulfonique, et on a chauffé le tout à reflux pendant 1 h. Après avoir refroidi à température ambiante, on a lavé avec 100 ml de NaOH à 10%. On a concentré au rotavapeur et distillé sur résidu pour obtenir 670 g de 2-(3,3-diméthyl-1-cyclohexyl)-2-méthyl-1,3-dioxolane.

P.éb. 53-59°/8 Pa ; rend.: 97,3%.

On a chauffé, sous azote et agitation, une solution de 212 g (1 mole) du cétal susmentionné dans 500 ml de toluène à 80° et introduit une solution de 170 g (1,2 mode) de DIBAH dans le toluène (≈ 800 ml de solution, 1,5 M). L'introduction a duré environ 2 h, avec une forte exothermie de la réaction au départ. On a laissé agiter pendant 1 h à 100°, ensuite refroidi, versé sur glace et acidifié avec HCl à 10%. Après lavage à neutralité, concentration et distillation sur résidu, on a obtenu 206 g d'un mélange de 5-(3,3-diméthyl-1-cyclohexyl)-4-oxa-2-hexanol et de 4-(3,3-diméthyl-1-cyclohexyl)-2-méthyl-3-oxa-1-pentanol.

P.éb. 70-78°/2 Pa ; rend.: 97,2%.

Dans un réacteur sous agitation, on a chargé 103 g (0,48 mole) du mélange d'alcools susmentionné, 450 ml de toluène et 79 g (1 mole) de pyridine et ajouté goutte à goutte 54 g (0,5 mode) de chlorure de propionyle. On a laissé réagir 3 h à température ambiante. Le mélange réactionnel a été versé sur glace et lavé 2 fois avec HCl à 10%, NaOH à 10% à froid (2 fois) et saumure (2 fois). On a concentré et distillé sur résidu pour obtenir un mélange (126 g) des deux propionates désirés.

P.éb. 100-105°/1,5 Pa, rend.: 96,9%.

Les deux composés sous titre ont également été préparés individuellement à partir des acétates décrits dans l'Exemple 1, à l'aide d'une réduction de ces derniers suivie d'une re-estérification.

Données analytiques :

propionate de 4-(3,3-diméthyl-1-cyclohexyl)-1-méthyl-3-oxapentyle
(4 stéréoisomères)

RMN($^1$H,360MHz) : 0,87(s,3H) ; 0,90(s,3H) ; 1,06(d,J=6Hz,3H) ; 1,14(t,J=7Hz,3H) ; 1,22-(d,J=6Hz,3H) ; 2,31(q,J=7Hz,2H) ; 3,06(m,1H) ; 3,34(m,1H) ; 3,50(m,1H) ; 5,0-(m,1H) δ ppm

SM : 270($M^+$,0), 159, 139, 115, 83, 69, 57

propionate de 4-(3,3-diméthyl-1-cyclohexyl)-2-méthyl-3-oxapentyle
(4 stéréoisomères)

RMN($^1$H,360MHz) : 0,87(s,3H) ; 0,90(s,3H) ; 1,07(t,J=5Hz,3H) ; 1,05-1,18(d/t,9H) ; 2,35-(q,J=7,5Hz,2H) ; 3,12-3,22(m,1H) ; 3,6-3,7(m,1H) ; 3,94-5,09(m,2H) δ ppm

SM : 270($M^+$,0), 183, 159, 139, 115, 83, 69, 57, 41

Les propriétés odorantes de ces deux composés et de leurs mélanges sont décrites dans l'introduction.

Exemple 3

Préparation d'acétate de 4-(3,3-diméthyl-1-cyclopentyl)-1-méthyl-3-oxapentyle et d'acétate de 4-(3,3-diméthyl-1-cyclopentyl)-2-méthyl-3-oxapentyle

Un mélange de ces deux composés a été préparé en utilisant 1-(3,3-diméthyl-1-cyclopentyl)-1-éthanone et 1,2-propanediol dans la réaction de cétalisation et anhydride acétique dans l'estérification, suivant le procédé décrit dans l'Exemple 1a) ou 2 et maintenant les autres réactifs décrits inchangés.

Données analytiques du mélange susmentionné :

RMN($^1$H,360MHz) : 0,95(s,3H) ; 1,0(s,3H) ; 1,06-1,17(d,6H) ; 2,03 et 2,05(2s,3H) ; 3,10-3,70(m,2H) ; 3,94-5,03(m,2H) δ ppm

SM : 242($M^+$,0), 145, 125, 109, 101, 69, 43

Propriétés odorantes : ce mélange possédait une note odorante nettement moins musquée, plus dure, plus poire et moins ambrette que celle des composés décrits dans l'Exemple 2. Elle était aussi plus puissante en tête mais moins tenace en mouillette que cette dernière.

La 1-(3,3-diméthyl-1-cyclopentyl)-1-éthanone de départ a été préparée ainsi : dans un ballon de 250 ml maintenu sous azote, on a chargé 2,1 g (0,088 mole) de Mg, auquel on a ajouté quelques ml d'éther pour le recouvrir, et introduit une dizaine de gouttes de $CH_3I$ pour démarrer la réaction. Après le démarrage de celle-ci, on a introduit le restant de 11,9 g de $CH_3I$ (0,084 mole) dans 30 ml d'éther éthylique en maintenant un léger reflux. On a ensuite chauffé à reflux pendant 1/2 h, refroidi à température ambiante et introduit 10 g (0,08 mode) de 4,4-diméthyl-1-cyclopentène-1-carbaldéhyde [préparé selon G. Magnusson et al., J. Org. Chem. 38, 1380 (1973)] en solution dans 30 ml d'éther. On a chauffé à reflux pendant 1 h. Après refroidissement, on a versé sur glace et HCl, lavé à neutralité avec NaCl sat., séché, filtré et concentré au rotavapeur. Après distillation au four à boules, on a obtenu 6 g (rend. : 53,6%) de 1-(4,4-diméthyl-1-cyclopentén-1-yl)-1-éthanol dont les données analytiques étaient :

RMN($^1$H,360MHz) : 1,08(s,3H) ; 1,09(s,3H) ; 1,26(d,J=7Hz,3H) ; 4,35(q,J=7Hz,1H) ; 5,45(s,1H) δ ppm

SM : 140($M^+$,11), 125(12), 122(23), 107(100), 91(63), 79(56), 43(21)

Dans un ballon de 250 ml sous $N_2$, on a chargé 13,2 g (60,8 mmole) de chlorochromate de pyridinium dans 100 ml de $CH_2Cl_2$ et on a refroidi à ≈ 10°. On a introduit goutte à goutte une solution de 6 g (43 mmole) de l'éthanol susmentionné dans 50 ml de $CH_2Cl_2$. On a laissé sous agitation pendant 3 h à température ambiante. On a versé le mélange réactionnel sur 200 ml d'éther et filtré sur colonne de $SiO_2$ (200 g). Après avoir concentré au rotavapeur et distillé au four à boules (100°/12x$10^2$ Pa), on a obtenu 3,2 g (rend. : 54%) de 1-(4,4-diméthyl-1-cyclopentén-1-yl)-1-éthanone dont les données analytiques étaient les suivantes :

RMN($^1$H,360MHz) : 1,09(s,6H) ; 2,3(s,3H) ; 2,37(s,4H) ; 6,64(s,1H) δ ppm

SM : 138($M^+$,53), 123(100), 95(72), 67(70), 43(98)

Dans un ballon de 250 ml, on a chargé 3 g (22 mmole) de la cétone insaturée susmentionnée, ajouté 100 ml d'éther acétique et environ 0,2 g de Pd/C 10%. Après avoir établi le vide, on a fait passer un courant de $H_2$ pendant 36 h (absorption ≈ 450 ml $H_2$). Le produit de la réaction a été filtré sur verre fritté, concentré au rotavapeur et distillé au four à boules (100°/9x$10^2$ Pa). On a obtenu 2,4 g (rend. 80%) de la cétone désirée, à savoir 1-(4,4-diméthyl-1-cyclopentyl)-1-éthanone dont les données analytiques étaient les suivantes :

RMN($^1$H,360MHz) : 0,98(s,3H) ; 1,02(s,3H) ; 2,15(s,3H) ; 3,02(quint,J=7Hz,1H) δ ppm

SM : 140($M^+$,7), 125(21), 97(53), 81(28), 71(41), 55(100), 43(87)

Exemple 4

Préparation de propionate de 4-(3,3-diméthyl-1-cyclopentyl)-1-méthyl-3-oxapentyle et de propionate de 4-(3,3-diméthyl-1-cyclopentyl)-2-méthyl-3-oxapentyle

On a préparé un mélange de ces deux composés selon le procédé décrit dans l'exemple 1a) ou 2, en utilisant 1-(3,3-diméthyl-1-cyclopentyl)-1-éthanone et 1,2-propanediol dans la réaction de cétalisation, et chlorure de propionyle dans celle d'estérification, les autres réactifs étant les mêmes que ceux décrits dans lesdits exemples.

Données analytiques :

RMN($^1$H,360MHz) : 0,95(s,3H) ; 1,0 et 1,01(2s,3H) ; 1,08-1,18(d+t,9H) ; 2,34(q, J=7,5Hz,2H) ; 3,1-3,8 et 4,0(m,4H) δ ppm

SM : 256($M^+$,0), 141, 125, 115, 109, 95, 81, 69, 57, 41

Propriétés odorantes : on retrouve dans la note odorante de ce mélange le joli caractère musqué et ambrette des composés préférés de l'invention décrits dans l'Exemple 2, avec plus de montant en tête. La

note de ce mélange est cependant plus fine et plus puissante en tête, mais a moins de volume et est moins tenace sur mouillette que celle desdits composés.

Exemple 5

Préparation d'isobutyrate de 4-(3,3-diméthyl-1-cyclohexyl)-1-méthyl-3-oxapentyle et d'isobutyrate de 4-(3,3-diméthyl-1-cyclohexyl)-2-méthyl-3-oxapentyle

On a préparé un mélange de ces deux composés selon le procédé décrit dans l'exemple 2, par réaction du mélange de 5-(3,3-diméthyl-1-cyclohexyl)-4-oxa-2-hexanol et 4-(3,3-diméthyl-1-cyclohexyl)-2-méthyl-3-oxa-1-pentanol avec chlorure d'isobutyrile dans l'estérification.

Données analytiques :

P.éb. 120°/20 Pa

RMN($^1$H,360MHz) : 0,87(s,3H) ; 0,90(s,3H) ; 1,07 et 1,17(d,12H) ; 2,55(m,1H) ; 3,1-4,05(m,4H) δ ppm

SM : 284($M^+$,0), 139, 129(100), 83, 71, 55, 43

Propriétés odorantes : ce mélange développait une odeur musquée, boisée, ambrée, ambrette relativement faible.

Exemple 6

Préparation de propionate de 4-(3,3-diméthyl-1-cyclohexyl)-2,2-diméthyl-3-oxapentyle et de propionate de 4-(3,3-diméthyl-1-cyclohexyl)-1,1-diméthyl-3-oxapentyle

On a procédé comme il est décrit dans l'exemple 2, en utilisant 10 g de cycladémone et un léger excès de 2-méthyl-1,2-propanediol [préparé à partir d'hydroxyacétone (Fluka) et $CH_3MgI$; RMN($^1$H,360MHz) : 1,19(s,6H) ; 3,4(s,2H) δ ppm ; SM : 90($M^+$,0), 75(17), 59(100), 57(25), 43(31)] dans du cyclohexane, avec 0,1 g d'acide p-toluènesulfonique. On a obtenu 14,3 g de 2-(3,3-diméthyl-1-cyclohexyl)-2,4,4-triméthyl-1,3-dioxolane (rend. : 97%).

P.éb. 50°/40 Pa

RMN($^1$H,360MHz) : 0,88(s,3H) ; 0,92(s,3H) ; 1,265/1,27(2s,3H) ; 1,28(s,3H) ; 1,33(s,3H) ; 3,27(m,2H) δ ppm

SM : 226($M^+$,0), 211(1), 168(1), 154(1), 139(2), 115(100), 69(23), 55(15), 43(61)

Ce cétal a été traité de façon analogue à celle décrite dans l'Exemple 2 avec 100 ml de solution de DIBAH (2 M dans le toluène). On a obtenu 13,2 g (rend. : 90%) d'un mélange de 5-(3,3-diméthyl-1-cyclohexyl)-2-méthyl-4-oxa-2-hexanod (73%) et de 4-(3,3-diméthyl-1-cyclohexyl)-2,2-diméthyl-3-oxa-1-pentanol (27%).

P.éb. 40-75°/20 Pa.

Ces alcools ont été séparés par chromatographie en phase préparative et ils consistaient chacun en un mélange racémique de deux isomères optiquement actifs.

Données analytiques :

5-(3,3-diméthyl-1-cyclohexyl)-2-méthyl-4-oxa-2-hexanol

RMN($^1$H,360MHz) : 0,89(s,3H) ; 0,91(s,3H) ; 1,08(d,J=6Hz,3H) ; 1,20(s,3H) ; 1,21(s,3H) ; 3,12(m,2H) ; 3,32(m,1H) δ ppm

SM (2 isomères) : isomère 1 : 228($M^+$,0), 169(5), 139(25), 123(26), 117(40), 83(74), 73(98), 59(100), 41(29)

isomère 2 : 228($M^+$,0), 170(5), 139(21), 123(25), 117(41), 83(69), 73(100), 59(95), 41(26)

4-(3,3-diméthyl-1-cycdohexyl)-2,2-diméthyl-3-oxa-1-pentanol

RMN($^1$H,360MHz) : 0,86/0,865(2s,3H) ; 0,90(s,3H) ; 1,055/1,06(2d,J=6Hz,3H) ; 1,135(s,3H) ; 1,15/1,155(2s,3H) ; 3,5(s,2H) ; 3,7(m,1H) δ ppm

SM (2 isomères) : isomère 1 : 228($M^+$,0), 197(10), 139(96), 117(27), 97(25), 83(96), 73(100), 55(42), 41(25)

isomère 2 : 228($M^+$,0), 197(10), 139(99), 117(31), 97(24), 83(100), 73(99), 55(50), 41(29)

Le mélange de ces deux alcools (10 g) dans 30 ml de pyridine a été traité avec chlorure de propionyle, de façon analogue à celle décrite dans l'Exemple 2, pour fournir un mélange des deux propionates désirés (11,2 g; rend. : 90% ; P. éb. 160°/15 Pa).

Les deux propionates ont été séparés par chromatographie en phase préparative.

Données analytiques :

propionate de 4-(3,3-diméthyl-1-cyclohexyl)-1,1-diméthyl-3-oxapentyle

RMN($^1$H,360MHz) : 0,87(s,3H) ; 0,89(s,3H) ; 1,05(d,J = 6Hz,3H) ; 1,08(t,J = 7Hz,3H) ; 1,42(s,6H) ; 2,225(q,J = 7Hz,2H) ; 3,07(hext.,J = 7Hz,1H); 3,48(dd,$J_1$ = 4,3, $J_2$ = 9,4Hz) ; 3,57-(dd,$J_1$ = 4,3Hz, $J_2$ = 9,4Hz) δ ppm

SM (2 isomères) : isomère 1 : 284($M^+$,0), 210(2), 155(2), 139(18), 129(34), 83(73), 72(79), 57(100), 41(38)

isomère 2 : 284($M^+$,0), 210(2), 155(3), 139(18), 129(39), 83(75), 72(73), 57(100), 41(23)

Propriétés odorantes : odeur faible, moins intéressante que celle de son isomère ci-après.

propionate de 4-(3,3-diméthyl-1-cyclohexyl)-2,2-diméthyl-3-oxapentyle

RMN($^1$H,360MHz) : 0,86/0,865(2s,3H) ; 0,895/0,90(2s,3H) ; 1,05/1,055(2d,J = 6Hz, 3H) ; 1,16-(t,J = 7Hz,3H) ; 1,18(s,6H) ; 2,37(q,J = 7Hz,2H) ; 3,37(m,1H) ; 3,94(s divisé,2H) δ ppm

SM (2 isomères) : isomère 1 : 284($M^+$,0), 197(2), 139(28), 129(78), 83(41), 69(32), 57(100), 41(22)

isomère 2 : 284($M^+$,0), 197(2), 139(27), 129(82), 83(43), 69(32), 57(100), 41(22)

Propriétés odorantes : décrites dans l'introduction.

Exemple 7

Préparation de propionate de (1'RS,4RS)-4-(3',3'-diméthyl-1'-cyclohexyl)-2,2-diméthyl-3-oxapentyle

Une solution de 2,5 g de (Z)-2-(3,3-diméthyl-1-cyclohexylidène)-1-éthanol [voir, par exemple, A. Gutmann et al., J. Chem. Ecology 7, 919 (1981)] dans le THF (tetrahydrofurane) a été ajoutée goutte à goutte, sous agitation et argon à un mélange de $LiAlH_4$ (2 g) et dichlorure de titanocène (Fluka, 4 pointes de spatule) dans du THF absolu à -40 °C. Le mélange réactionnel a été laissé sous agitation à température ambiante pendant le weekend. Après un lavage à l'eau (2 ml), NaOH à 15% (2 ml) et de nouveau eau (6 ml) pour hydroliser et filtration, on a obtenu 1,9 g de (Z)-3-éthylidène-1,1-diméthylcyclohexane qui a été utilisé dans la suite de la synthèse. Une petite quantité a été distillée au four à boules pour analyse.

RMN($^1$H,360MHz) : 0,88(s,6H) ; 1,54(d,J = 6Hz,3H) ; 5,24(q,J = 6Hz,1H) δ ppm

Le restant de ce produit a été ajouté à une solution 1 molaire de $B_2H_6$ dans le THF et le mélange a été agité à température ambiante pendant 24 h. On a ensuite ajouté 50 ml de NaOH à 15% et 20 ml de perhydrol et laissé agiter pendant la nuit. Après extraction à l'éther, lavage à la saumure, concentration et distillation (P. éb. 100 °/20 Pa), on a obtenu 0,7g de produit dont le GC a montré qu'il s'agissait d'un produit contenant 82% de (1RS,1'RS)-1-(3',3'-diméthyl-1'-cyclohexyl)-1-éthanol et environ 18% de son isomère (1RS,1'SR).

RMN($^1$H,360MHz) : 0,885 et 0,915(2s,6H) ; 1,15(d,J = 6Hz,3H) ; 1,85(d large,1H) ; 3,5(m,1H) δ ppm

RMN($^{13}$C) : 20,6(q) ; 22,1(t) ; 24,8(q) ; 28,1(t) ; 30,7(s) ; 33,8(q) ; 39,1(t) ; 40,9(d) ; 42,0(t) ; 72,4(d) δ ppm

SM : isomère (1RS,1'RS) : 154($M^+$,1), 138(4), 123(30), 112(33), 97(73), 81(39), 69(100), 55(46), 45(33), 41(42)

isomère (1RS,1'SR) : 154($M^+$,1), 138(6), 123(30), 112(30), 97(70), 81(37), 69(100), 55(47), 45(30), 41(41)

On a ajouté à 700 mg de l'alcool ainsi préparé, en solution dans 10 ml de toluène, 1 ml de isobuténoxyde (BASF) et 5 gouttes de $BF_3.(C_2H_5)_2O$, à -10° et sous agitation. On a laissé réagir pendant 4 h à température ambiante et ensuite soumis le produit de la réaction au traitement usuel. Un GC a montré qu'il restait encore 40% de produit de départ et on a donc répété la procédure et laissé en repos pendant la nuit à température ambiante. Le mélange a ensuite été estérifié avec anhydride propionique dans la pyridine (1 nuit) pour fournir un produit contenant 84% de propionate de (1'RS,4RS)-4-(3',3'-diméthyl-1'-cyclohexyl)-2,2-diméthyl-3-oxapentyle et 16% de son isomère (1'SR,4RS).

ISOMERE MAJORITAIRE

RMN($^1$H,360MHz) : 0,86 et 0,90(2s,6H) ; 1,055(d,J = 6Hz,3H) ; 1,155(t,J = 7Hz,3H) ; 1,18(s,6H) ; 1,82(d large,1H) ; 2,36(d,J = 7Hz,2H) ; 3,37(quint.,J = 6Hz,1H) ; 3,95(s divisé,2H) δ ppm

RMN($^{13}$C) : 174,3(s) ; 73,9(s) ; 71,9(d) ; 70,5(t) ; 41,8(t) ; 40,6(d) ; 39,5(t) ; 33,7(q) ; 30,7(s) ; 29,4(t) ; 27,7(t) ; 24,8(q) ; 24,2(q) ; 23,9(q) ; 22,4(t) ; 19,9(q) ; 9,2(q) δ ppm

SM : 284($M^+$,0), 197(2), 139(27), 129(82), 83(43), 69(32), 57(100), 41(22)

ISOMERE MINORITAIRE

RMN($^1$H,360MHz) : 0,86 et 0,90(2s,6H) ; 1,05(d,J = 6Hz,3H) ; 1,16(t,J = 7Hz,3H) ; 1,165/1,175(2s,6H) ;

| | |
|---|---|
| | 1,66(d large,1H) ; 2,365(d,J=7Hz,2H) ; 3,37(quint., J=6Hz,1H) ; 3,94(s divisé,2H) δ ppm |
| RMN($^{13}C$) : | 174,3(s) ; 73,8(s) ; 71,9(d) ; 70,5(t) ; 42,3(t) ; 40,5(d) ; 39,5(t) ; 33,7(q) ; 30,7(s) ; 28,4(t) ; 27,7(t) ; 24,7(q) ; 24,2(q) ; 23,8(q) ; 22,4(t) ; 19,7(q) ; 9,2(q) δ ppm |
| SM : | 284($M^+$,0), 197(2), 139(28), 129(78), 83(41), 69(32), 57(100), 41(22) |

Propriétés odorantes : poire, musqué ambrette, très agréable.

Exemple 8

Préparation du propionate de (-)-(1'S,4R)-4-(3',3'-diméthyl-1'-cyclohexyl)-2,2-diméthyl-3-oxapentyle

On a refroidi à l'aide d'un bain de glace et sous agitation un mélange de 146 g (0,93 mode) de (-)-(1R,1'S)-1-(3',3'-diméthyl-1'-cyclohexyl)-1-éthanol ["(-)-cyclademol", origine : DRT, France ; $[\alpha]^{20}_D$ = -11°, contient 13% de son diastéréomère (1R,1'R)], isobutylène oxyde (BASF) et 100 ml de cyclohexane. On a ajouté au mélange 1 ml de $BF_3.(C_2H_5)_2O$ et, 30 min après, encore 1 ml de $BF_3.(C_2H_5)_2O$. On a laissé réagir le mélange refroidi pendant 3 h et ensuite on a lavé avec NaOH dilué, concentré et distillé sur colonne garnie de 50 cm. Le produit ainsi obtenu (3,8 g), contenant 76% de (1'S,4R)-4-(3',3'-diméthyl-1'-cyclohexyl)-2-méthyl-4-oxa-2-éthanol, a été chauffé à 100° avec 10 ml d'anhydride propionique pendant 6 h et ensuite distillé au four à boules pour fournir 4,4 g du propionate désiré.

P.éb. 130° (bain)/10 Pa; rend.: 94%

$\alpha^{20}_D$ = -7,12° (pur)

| | |
|---|---|
| RMN($^1H$,360MHz) : | 0,86 et 0,90(2s,6H) ; 1,05(d,J=6Hz,3H) ; 1,16(t,J=7Hz,3H) ; 1,18(s,6H) ; 1,66(d large,1H) ; 2,37(d,J=7Hz,2H) ; 3,37(quint.,J=6Hz,1H) ; 3,94(s divisé,2H) δ ppm |
| SM : | 284($M^+$,0), 197(2), 139(28), 129(78), 83(41), 69(32), 57(100), 41(22) |

Propriétés odorantes : agréablement musqué et ambrette, poire, floral.

Exemple 9

Préparation du propionate de (+)-(1'R,4S)-4-(3'3'-diméthyl-1'-cyclohexyl)-2,2-diméthyl-3-oxapentyle

On a fait réagir 6,2 g de (+)-(1'R,1S)-1-(3',3'-diméthyl-1'-cyclohexyl)-1-éthanol [préparé à partir de (+)-β-citronellène (Fluka) selon de procédé décrit par H.R. Ansari, Tetrahedron 29 1559 (1973), $[\alpha]^{20}_D$ = +11,8°] avec isobutylène oxyde (BASF). Après distillation on a obtenu 3,3 g d'alcool de départ et 2 g de produit intermédiaire désiré. On a fait réagir ce dernier avec anhydride propionique pour obtenir, après chromatographie en phase préparative le propionate désiré.

$\alpha^{20}_D$ = +6,5° (pur)

Les autres données analytiques étaient identiques à celles du composé décrit dans l'Exemple 8.

Propriétés odorantes : musqué, ambrette, poire.

Exemple 10

Préparation d'une composition parfumante

On a préparé une composition parfumante de base par mélange des ingrédients suivants :

| Ingrédients | Parties en poids |
|---|---|
| Acetate d'hexyle | 50 |
| Acétate de polyméthylol 1) | 1500 |
| Acétate TCD 2) | 1500 |
| Cinnamate de méthyle | 250 |
| γ-Décalactone à 10%* | 200 |

| | |
|---|---|
| γ-Dodécalactone | 50 |
| β-Damascone à 50%* | 250 |
| Isopentyrate 3) | 100 |
| Myroxyde ® 4) | 100 |
| Veloutone 5) | 700 |
| Bourgeonal 6) à 10%* | 300 |
| Lilial ® 7) à 10%* | 2700 |
| n-Octanal à 1%* | 100 |
| Mayol ® 8) | 200 |
| Total | 8000 |

* dans le dipropylène glycol (DIPG)

1) acétate de tétraméthylnonyle ; origine : Firmenich SA, Genève, Suisse
2) acétate de (tricyclo[5.2.1.$0^{2,6}$]déc-4-yl)méthyle : origine : Firmenich SA, Genève, Suisse
3) isobutyrate de 1,3-diméthyl-3-butényle ; origine : Firmenich SA, Genève, Suisse
4) mélange d'isomères de l'époxyde ocimène ; origine : Firmenich SA, Genève, Suisse
5) 2,2,5-triméthyl-5-pentyl cyclopentanone ; origine : Firmenich SA, Genève, Suisse
6) 3-(4-tert-butyl-1-phényl)propanal ; origine : Quest International
7) 3-(4-tert-butyl-1-phényl)-2-méthylpropanal ; origine : L. Givaudan, Vernier, Suisse
8) 1-hydroxyméthyl-4-isopropyl cyclohexane ; origine : Firmenich SA, Genève, Suisse

Lorsqu'on a ajouté à cette composition de base 2000 parties en poids de l'un des composés préférés de l'invention, décrits dans les Exemples 2 ou 6 à 9, on a obtenu une nouvelle composition dont l'odeur possédait une nette connotation poire William. Par ailleurs, la nouvelle composition développait la note ambrette caractéristique de la poire, qui avait été totalement absente de la composition de base. Cet effet olfactif était particulièrement marqué et élégant lorsque l'on avait ajouté, à la composition, le composé de l'invention décrit à l'Exemple 9.

Exemple 11

Préparation d'une composition parfumante pour détergent en poudre

On a préparé une composition parfumante de base pour un détergent en poudre par mélange des ingrédients suivants :

| Ingrédients | Parties en poids |
|---|---|
| Acétate de citronellyle | 200 |
| Aldéhyde amylcinnamique | 1000 |
| Aldéhyde hexylcinnamique | 2000 |
| Ambrox ® 1) DL à 10%* | 100 |
| Acétate d'isononyle | 200 |
| Acétate de verdyle | 400 |
| Propionate de verdyle | 500 |
| Aldéhyde Intreleven 2) à 10%* | 100 |
| Aldéhyde 13-13 3) à 10%* | 200 |
| Coumarine | 100 |
| Lilial ® 4) | 1000 |
| Acétate de 4-tert-butyl-cyclohexyle 5) | 1300 |
| Fleuramone ® 6) | 200 |
| 3-Méthyl-5-phényl-1-pentanol 7) | 500 |
| Salicylate de benzyle | 700 |
| Tétrahydro muguol 8) | 400 |
| α-Damascone à 10%* | 250 |
| Polywood ® 9) | 150 |
| Isoraldéine ® 10) 70 P | 300 |
| Vertofix coeur 11) | 400 |
| Total | 10000 |

* dans le DIPG

1) tétraméthyl perhydronaphtofuranne racémique; origine : Firmenich SA, Genève, Suisse

2) mélange d'isomères de l'undécénal ; origine : International Flavors & Fragrances, USA

3) n-/i-Tridécanal ; origine : Henkel

4) voir Exemple 10

5) riche en isomère cis ; origine : Firmenich SA, Genève, Suisse

6) 2-heptyl-1-cyclopentanone ; origine : International Flavors & Fragrances, USA
7) origine : Firmenich SA, Genève, Suisse
8) mélange isomérique ; origine : International Flavors & Fragrances, USA
9) acétate de perhydro-5,5,8a-triméthyl-2-naphtyle ; origine : Firmenich SA, Genève, Suisse
10) iso-méthyl ionone ; origine : L. Givaudan, Vernier, Suisse
11) origine : International Flavors & Fragrances, USA

Lorsqu'on a ajouté à cette composition de base pour détergent en poudre 1000 parties en poids de l'un des composés de l'invention préparés selon les Exemples 2 ou 6 à 9, on a obtenu une nouvelle composition dont la note odorante avait plus de volume et de puissance, ainsi qu'un net aspect musqué et légèrement fruité.
Cet effet olfactif était encore plus perceptible sur du linge lavé avec un détergent en poudre parfumé à l'aide de cette nouvelle composition. L'aspect musqué de la note odorante de la composition nouvelle selon l'invention, que l'on sentait également sur le linge ainsi traité, était tout à fait différent des notes musquées typiquement conférées par les composés de type musqué disponibles sur le marché, les composés préférés selon l'invention permettant d'obtenir un effet odorant plus poudré-ambrette que les composés macrocycles ou indoliques à odeur musquée connus. Comme dans le cas de l'Exemple précédent, le composé préféré selon l'invention décrit à l'Exemple 9 permettait d'obtenir au mieux l'effet olfactif décrit ci-dessus.

**Revendications**

**1.** Composé de formule

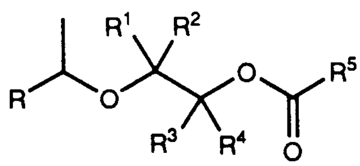

(I)

dans laquelle le symbole R représente un radical 3,3-diméthyl-1-cyclopentyle ou 3,3-diméthyl-1-cyclohexyle, le symbole $R^5$ représente un radical alkyle saturé de $C_1$ à $C_3$, de chaîne linéaire ou ramifiée et, soit $R^1$ et $R^2$ représentent chacun un atome d'hydrogène, $R^3$ et/ou $R^4$ représentant un radical méthyle, soit $R^3$ et $R^4$ représentent chacun un atome d'hydrogène, $R^1$ et/ou $R^2$ représentant alors un radical méthyle.

**2.** Mélange de composés selon la revendication 1, sous forme des isomères de formule

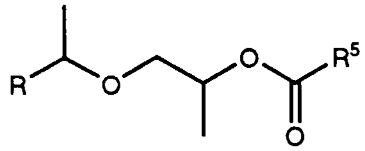

et

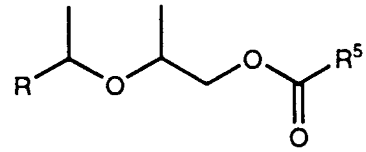

(Ia) (Ib)

dans lesquelles les symboles R et $R^5$ ont le sens indiqué à la formule (I).

3. Mélange de composés selon la revendication 1, sous forme des isomères de formule

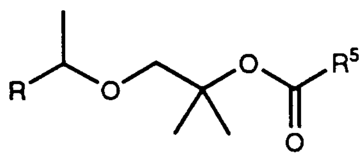

(Ic)

et

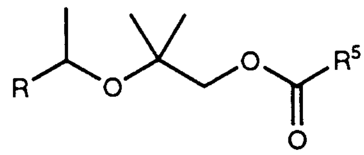

(Id)

dans lesquelles les symboles R et $R^5$ ont le sens indiqué à la formule (I).

4. Propionate de 4-(3,3-diméthyl-1-cyclohexyl)-2,2-diméthyl-3-oxapentyle sous forme racémique ou sous forme de l'un de ses isomères optiquement actifs de formule

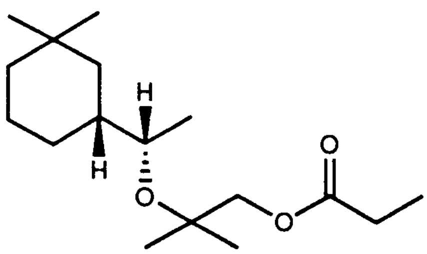

(Ie)
(+)-(1'R,4S)

ou

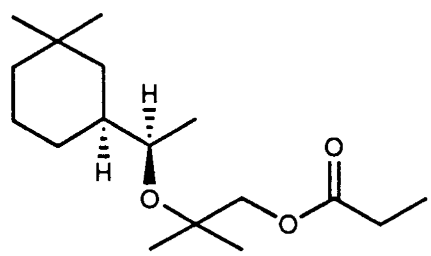

(If)
(-)-(1'S,4R)

5. Propionate de 4-(3,3-diméthyl-1-cyclohexyl)-2-méthyl-3-oxapentyle, propionate de 4-(3,3-diméthyl-1-cyclohexyl)-1-méthyl-3-oxapentyle ou tout mélange de ces deux composés.

6. Utilisation, à titre d'ingrédient parfumant, d'un composé ou d'un mélange selon l'une des renvendications 1 à 5, pour la préparation de compositions parfumantes ou d'articles parfumés.

7. Composition parfumante ou article parfumé résultant de l'utilisation selon la revendication 6.

8. A titre d'article parfumé selon la revendication 7, un parfum ou une eau de toilette, un savon, un gel de bain ou douche, un shampoing ou autre produit de traitement capillaire, un désodorisant corporel ou d'air ambiant, un détergent ou un revitalisant textile ou un produit d'entretien.

9. Procédé pour la préparation d'un composé de formule (I) définie à la revendication 1, ou d'un mélange de composés selon la revendication 2 ou 3, caractérisé en ce qu'on traite avec un agent d'estérification approprié un hydroxy-éther de formule

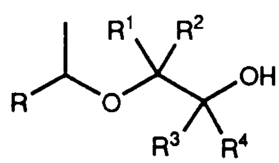

(II)

dans laquelle les symboles R, et $R^1$ à $R^4$ ont le sens indiqué à la formule (I), ou un mélange d'isomères structurels de formule (II).

10. Hydroxy-éther de formule (II) telle que définie à la revendication 9, ou tout mélange d'isomères structurels de formule (II).

## Claims

1. A compound of formula

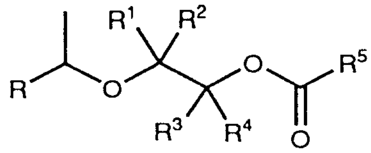

( I )

wherein symbol R represents a 3,3-dimethyl-1-cyclopentyl or a 3,3-dimethyl-1-cyclohexyl radical, symbol $R^5$ represents a saturated, linear or branched, $C_1$ to $C_3$ alkyl radical and, either $R^1$ and $R^2$ stand each for a hydrogen atom, $R^3$ and/or $R^4$ representing a methyl radical, or $R^3$ and $R^4$ represent each a hydrogen atom, $R^1$ and/or $R^2$ representing then a methyl radical.

2. A mixture of compounds according to claim 1, in the form of isomers of formula

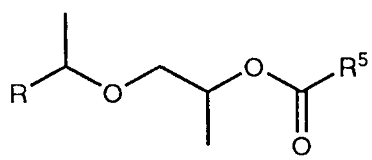

and

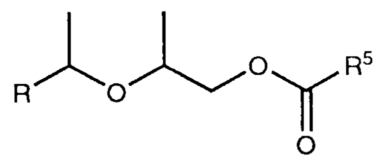

( Ia ) ( Ib )

wherein symbols R and $R^5$ have the meaning indicated in formula (I).

3. A mixture of compounds according to claim 1, in the form of isomers of formula

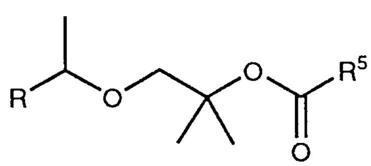

and

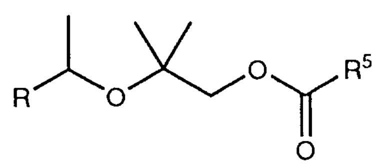

( Ic ) ( Id )

wherein symbols R and $R^5$ have the meaning indicated in formula (I).

4. 4-(3,3-Dimethyl-1-cyclohexyl)-2,2-dimethyl-3-oxapentyl propanoate in racemic form or in the form of one of its optically active isomers of formula

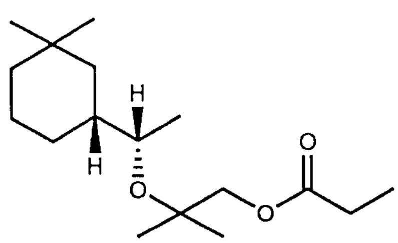

or

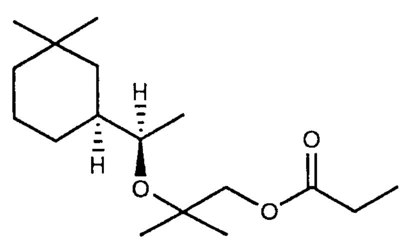

( Ie ) (+)-(1'R,4S) ( If ) (-)-(1'S,4R)

5. 4-(3,3-Dimethyl-1-cyclohexyl)-2-methyl-3-oxapentyl propanoate, 4-(3,3-dimethyl-1-cyclohexyl)-1-methyl-3-oxapentyl propanoate or any mixture of these two compounds.

6. Use as a perfuming ingredient of a compound or a mixture according to any one of claims 1 to 5, for the preparation of perfuming compositions or perfumed articles.

7. A perfuming composition or a perfumed article resulting from the use according to claim 6.

8. As a perfumed article according to claim 7, a perfume or cologne, a soap, a shower or bath gel, a shampoo or other hair-care product, a body or air deodorant, a detergent or a fabric softener, or a household product.

9. A process for the preparation of a compound of formula (I) as defined in claim 1, or of a mixture of compounds according to claim 2 or 3, characterized in that a hydroxy-ether of formula

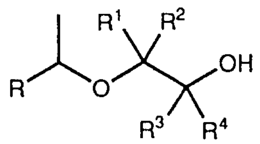

(II)

wherein symbols R and $R^1$ to $R^4$ are defined as in formula (I), or a mixture of appropriate structural isomers of formula (II), is treated with an appropriate esterification agent.

10. Hydroxy-ether of formula (II) as defined in claim 9, or any mixture of structural isomers of formula (II)

**Patentansprüche**

1. Verbindung der Formel

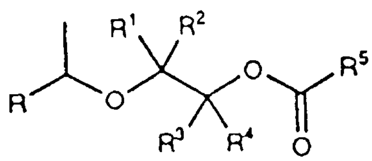

(I)

worin R für einen 3,3-Dimethyl-1-cyclopentyl- oder 3,3-Dimethyl-1-cyclohexyl-Rest steht, das Symbol $R^5$ einen gesättigten Alkylrest von $C_1$ bis $C_3$ mit gerader oder verzweigter Kette bedeutet und entweder $R^3$ und/oder $R^4$ einen Methylrest bedeuten, wenn $R^1$ und $R^2$ jeweils für ein Wasserstoffatom stehen oder $R^3$ und $R^4$ jeweils ein Wasserstoffatom bedeuten, wenn $R^1$ und/oder $R^2$ für einen Methylrest stehen.

2. Gemisch von Verbindungen gemäss Anspruch 1, in Form von Isomeren der Formel

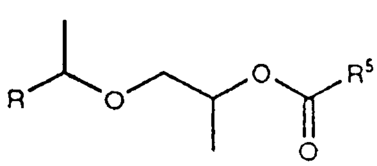

und

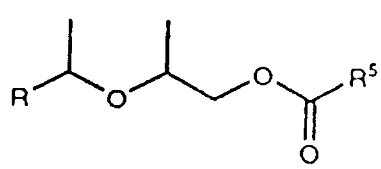

(Ia) (Ib)

worin die Symbole R und $R^5$ die für Formel (I) angegebene Bedeutung besitzen.

**3.** Gemisch von Verbindungen gemäss Anspruch 1, in Form von Isomeren der Formel

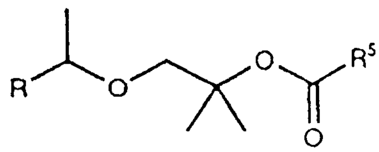

und

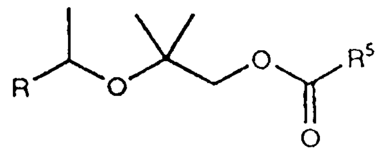

( Ic ) ( Id )

worin die Symbole R und $R^5$ die für Formel (I) angegebene Bedeutung besitzen

**4.** 4-(3,3-Dimethyl-1-cyclohexyl)-2,2-dimethyl-3-oxapentyl-propionat in racemischer Form oder in Form eines seiner optisch aktiven Isomeren der Formel

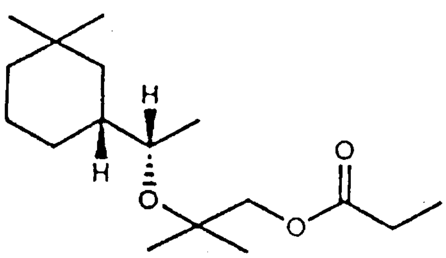

oder

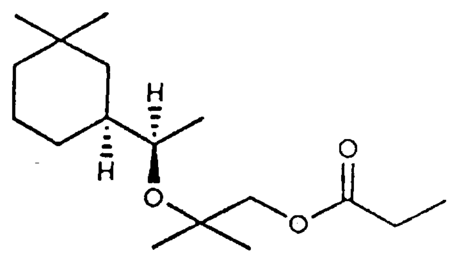

( Ie ) (+)-(1'R,4S)

( If ) (-)-(1'S,4R)

**5.** 4-(3,3-Dimethyl-1-cyclohexyl)-2-methyl-3-oxapentyl-propionat, 4-(3,3-Dimethyl-1-cyclohexyl)-1-methyl-3-oxapentyl-propionat oder alle Gemische dieser beiden Verbindungen.

**6.** Verwendung einer Verbindung oder eines Gemisches gemäss einem der Ansprüche 1 bis 5, als Riechstoffbestandteil für die Herstellung von Riechstoffkompositionen oder parfümierten Artikeln.

**7.** Riechstoffkomposition oder parfümierter Artikel, erhalten durch die Verwendung gemäss Anspruch 6.

**8.** Als parfümierter Artikel gemäss Anspruch 7, ein Parfum, oder ein Toilettwasser, eine Seife, ein Bade- oder Duschgel, Schampon oder ein anderes Haarbehandlungsmittel, ein Desodorierungsmittel für den Körper oder die Raumluft, ein Reinigungsmittel oder ein Textilauffrischungsmittel oder ein Pflegeprodukt.

**9.** Verfahren zur Herstellung einer im Anspruch 1 definierten Verbindung der Formel (I) oder eines Gemisches von Verbindungen gemäss dem Anspruch 2 oder 3, dadurch gekennzeichnet, dass man mit einem geeigneten Veresterungsmittel einen Hydroxyether der Formel

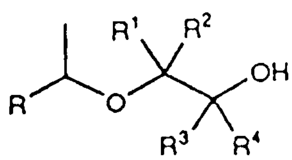

( II )

worin die Symbole R und $R^1$ bis $R^4$ die für die Formel (I) angegebene Bedeutung besitzen oder ein Gemisch von Strukturisomeren der Formel (II) behandelt.

**10.** Hydroxyether der Formel (II), wie im Anspruch 9 definiert, oder alle Gemische von Strukturisomeren der Formel (II).